# EUROPEAN PATENT APPLICATION

(11) **EP 3 192 801 A1**
(43) Date of publication of application: **19.07.2017**
(21) Application number: 17152057.0
(22) Date of filing: 18.01.2017
(51) Int. Cl.: C07K 7/08, C07K 19/00, G01N 33/68

(54) **PEPTIDES DERIVATIVES**

(30) Priority: 18.01.2016 GB 201600903
(71) Applicant: Bicycle Therapeutics Limited, Babraham Research Campus Cambridge CB22 3AT (GB)
(72) Inventor: TEUFEL, Daniel, Cambridge, Cambridgeshire CB22 3AT (GB); Baldassarre, Leonardo, Cambridge, Cambridgeshire CB22 3AT (GB)
(74) Representative: Maschio, Antonio

(57) **Abstract**

A compound comprising at least one looped peptide structure attached via at least two oxoether linkages to a scaffold. Preferably the looped peptide structure is attached to the scaffold via at least three oxoether linkages. Also provided is a method of making a compound comprising at least one looped peptide structure attached via at least two oxoether linkages to a scaffold, the method comprising: providing a peptide having at least two amino acid residues having at least one -OH group in a side chain thereof; optionally converting said -OH groups to leaving groups; providing a scaffold molecule having at least two reactive sites for forming oxoether linkages with the said at least two amino acid residues; and forming said oxoether linkages between the peptide and the scaffold molecule.

## Description

### Technical Field

The present invention relates to peptides whose structure is constrained by covalently linking to a scaffold moiety which provides a more structured backbone, imparting a conformation to the peptide. In particular, the invention relates to novel chemistries for forming two or more bonds between a peptide and a scaffold molecule.

### Background of the Invention

Different research teams have previously tethered peptides to scaffold moieties by forming two or more thioether bonds between cysteine residues of the peptide and suitable functional groups of a scaffold molecule. For example, methods for the generation of candidate drug compounds by linking cysteine-containing peptides to a molecular scaffold as for example tris(bromomethyl)benzene are disclosed in WO 2004/077062 and WO 2006/078161.

WO2011/018227 describes a method for altering the conformation of a first peptide derivative or group of peptide derivatives, each peptide derivative comprising at least two reactive groups separated by a loop sequence covalently linked to a molecular scaffold which forms covalent bonds with said reactive groups, to produce a second peptide derivative or group of peptide derivatives, comprising assembling said second derivative or group of derivatives from the peptide(s) and scaffold of said first derivative or group of derivatives, incorporating one of: (a) altering at least one reactive group; or (b) altering the nature of the molecular scaffold; or (c) altering the bond between at least one reactive group and the molecular scaffold; or any combination of (a), (b) or (c).

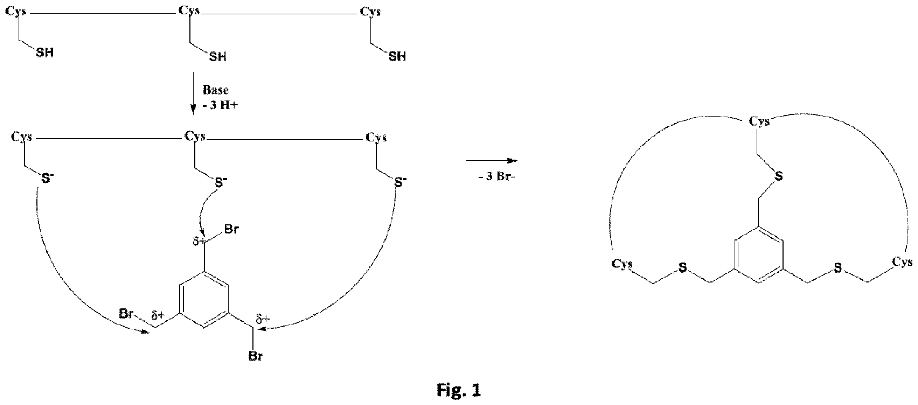

The advantage of utilising cysteine thiols for generating covalent thioether linkages in order to achieve cyclisation resides is their selective and biorthogonal reactivity. Thiol-containing linear peptides may be cyclised with a thiol-reactive scaffold compound such as 1, 3, 5 tris-bromomethylbenzene (TBMB) to form Bicyclic Peptides,and the resultant product contains three thioethers at the benzylic locations. The overall reaction of the linear peptide with TBMB to form a looped bicyclic peptide with thioether linkages is shown in Fig. 1.

In certain *in vivo* applications, and in the course of the manufacture of such thioether bonded peptides, the sulfur may represent a group that is susceptible to oxidation, forming in the first instance an *R* or *S* sulfoxide, and upon further oxidation, a sulfone:

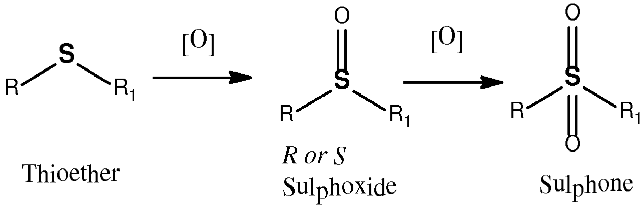

In the *in vivo* setting, these oxidised species may 1) alter the specificity of the (bicyclic) peptide to its target and its homologues, 2) unfavourably lower the affinity of the peptide to its target, lowering exposure, 3) complicate the interpretation of pharmacokinetics and pharmacodynamics *in vivo,* and 4) complicate the bioanalytical quantification *in vivo.*

In the manufacturing setting, the thioethers (as well as the thiol starting material) are sensitive to oxygen in the air, and special precautions are required to minimise formation of these oxidised species. Oxidised products result in lower yields and complications during purification and workup. Furthermore, upon preparation and storage of the formulated drug product for therapeutic or *in vivo* applications in general, or other engineering applications, thioethers are once again sensitive to oxidative processes and various precautions such as specialised formulations and protective atmospheres may be necessary.

A need therefore exists for improved chemistries for coupling peptides to scaffold moieties to form looped peptide structures employing suitable replacements of the thioether moiety.

### Summary of the Invention

The present inventors have found that replacement of the thioether linkages in a looped peptide by oxoether linkage offers an opportunity to generate looped peptide conjugates with improved oxidation stability.

Replacement of the thioether linkage with oxoether can be expected to result in retention of the biological properties of the molecule, such as affinity to the target and selectivity.

Thioether bonds with adjacent carbons display a bond angle of 90 degrees, with a bond length of 180 pm, while oxo-ether bonds display a bond angle of 110 degrees, with a bond length of 140 pm; thus, the dimensions and angles of thioether and oxoether linkages are comparable. Accordingly, Bicyclic compounds of the present invention should in principle present a fully functional and oxidation resistant replacement of the parent thioether Bicyclic Peptides.

Accordingly, in a first aspect the present invention provides a compound comprising at least one looped peptide structure attached via at least two oxoether linkages to a scaffold. Suitably, the looped peptide structure is attached to the scaffold via at least three oxoether linkages.

Suitably, the scaffold comprises a (hetero)aromatic or (hetero)alicyclic moiety. Suitably, the scaffold comprises a *tris*-substituted (hetero)aromatic or (hetero)alicyclic moiety, for example a tris-methylene substituted (hetero)aromatic or (hetero)alicyclic moiety. The (hetero)aromatic or (hetero)alicyclic moiety is suitably a six-membered ring structure, preferably *tris*-substituted such that the scaffold has a 3-fold symmetry axis. Thus, in embodiments, the scaffold is 1,3,5-*tris*-methylbenzene.

Suitably, the oxoether linkages are made through amino acid residues of the peptide having at least one -OH group in a side chain thereof. These amino acid residues may be naturally occurring amino acids such as serine or threonine, or non-natural amino acids such as N-methyl serine (other such non-natural amino acids will be apparent to the skilled reader, some of which are listed further below). Suitably, the oxoether linkages are made through the side-chain -OH of serine or threonine residues, and more suitably they are made through the side-chain -OH of serine residues.

In a second aspect, the present invention provides a method of making a compound comprising at least one looped peptide structure attached via at least two oxoether linkages to a scaffold, the method comprising: providing a peptide having at least two amino acid residues having at least one -OH group in a side chain thereof; providing a scaffold molecule having at least two reactive sites for forming oxoether linkages with the amino acid residues having at least one -OH group in a side chain thereof; and forming said oxoether linkages between the peptide and the scaffold molecule.

Suitably, the peptide has protecting groups on nucleophilic groups other than the -OH groups intended for forming the oxoether linkages.

In embodiments, the method of the invention comprises: synthesising a peptide having protecting groups on nucleophilic groups other than the -OH groups intended for forming the oxoether linkages and a second set of protecting groups on the -OH groups intended for forming oxoether linkages, wherein the protecting groups on the -OH groups intended for forming oxoether linkages can be removed under conditions different than for the protecting groups on the other nucleophilic groups, followed by treating the peptide under conditions selected to deprotect the -OH groups intended for forming oxoether linkages without deprotecting the other nucleophilic groups. For example, the protecting groups on the -OH groups intended for forming oxoether linkages may be more acid-labile than the protecting groups on the other nucleophilic peptide residues, whereby mild acid conditions can be used to deprotect these groups without deprotecting the other nucleophilic groups.

Suitably, the method of the invention comprises reacting, in a nucleophilic substitution reaction, the peptide having two or more -OH groups with a scaffold molecule having two or more leaving groups.

In alternative embodiments, the method comprises converting two or more -OH groups intended to form oxoether linkages to leaving groups on the partially protected peptide, followed by reacting the leaving group-activated peptide, in a nucleophilic substitution reaction, with a scaffold molecule having two or more -OH groups.

The nucleophilic substitution reactions may be performed in the presence of a base, for example where the leaving group is a conventional anionic leaving group. In other embodiments, the substitution reaction may be done under acid conditions, for example if the leaving group is 2,2,2-trichloroacetimidate. The nucleophilic substitution reactions may be suitably catalysed, for example with silver (I) oxide or with a phase transfer catalyst such as tetrabutylammonium iodide.

In yet other embodiments, the method comprises reacting the -OH groups on the peptide with acryloyl groups on the scaffold molecule. The acryloyl groups may, for example, be α,β-unsaturated amide or carboxylate groups on the scaffold molecule. The resulting α,β-addition reaction results in an oxoether linkage at the α- or β-carbon atom of the acryloyl group scaffold molecule, preferably at the β-carbon atom. Examples of a tris-acryloyl structures forming Bicyclic Peptides with linear thiol-containing peptides have been published (Shen et al, (2014) Angew Chem Int Ed Engl. 2014 Feb 3;53(6):1602-6). Thus, in embodiments of the peptide compounds of the invention, the scaffold comprises oxoether linkages at alpha or beta- carbon positions with respect to a carbonyl, situated on a (hetero)aryl scaffold or (hetero)alicyclic scaffold

In a further aspect, the present invention provides a library comprising a plurality of different compounds according to the invention. The different compounds may comprise different peptide sequences and/or different scaffolds. The library can be screened to identify compounds having a desired biological activity.

In a further aspect, the present invention provides a method for selecting a candidate drug compound, comprising: providing a library of compounds according to the above aspect of the invention, determining the binding of a target molecule to said compounds, and identifying a compound which maximally binds to said target molecule.

Screening may be carried out by analysis of individual molecules. Such methods are provided in WO 2004/077062, WO 2006/078161 and WO2011/018227.

In another aspect, the invention further provides a kit comprising at least a peptide derivative according to the present invention.

In a still further aspect, the present invention provides a composition comprising a peptide derivative of the present invention, and a pharmaceutically acceptable carrier, diluent or excipient.

Moreover, the present invention provides a method for the treatment of disease using a peptide derivative or a composition according to the present invention.

In a further aspect, the present invention provides a method for the diagnosis, including diagnosis of disease using a peptide derivative, or a composition according to the present invention. Thus in general the binding of an analyte to a peptide derivative may be exploited to displace an agent, which leads to the generation of a signal on displacement. For example, binding of analyte (second target) can displace an enzyme (first target) bound to the peptide derivative providing the basis for a binding assay, especially if the enzyme is held to the peptide derivative through its active site.

### Brief Description of the Drawings

Fig. 1 shows a reaction scheme for preparation of thioether-linked bicyclic peptide derivatives according to the prior art;
Fig. 2 shows a first reaction scheme for the preparation of oxoether-linked bicyclic peptide derivatives according to the present invention; and
Fig. 3 shows a second reaction scheme for the preparation of oxoether-linked bicyclic peptide derivatives according to the present invention.

### Detailed Description of the Invention

Unless defined otherwise, all technical and scientific terms used herein have the same meaning as commonly understood by those of ordinary skill in the art, such as in the arts of peptide chemistry, cell culture and phage display, nucleic acid chemistry and biochemistry. Standard techniques are used for molecular biology, genetic and biochemical methods (see Sambrook et al., Molecular Cloning: A Laboratory Manual, 3rd ed., 2001, Cold Spring Harbor Laboratory Press, Cold Spring Harbor, NY; Ausubel et al., Short Protocols in Molecular Biology (1999) 4th ed., John Wiley & Sons, Inc.), which are incorporated herein by reference.

The present invention provides a compound comprising at least one looped peptide structure attached via at least two oxoether linkages to a scaffold.

The compound of the invention therefore comprises at least one peptide loop, subtended between two oxoether linkages on a molecular scaffold.

Suitably, the looped peptide structure is attached to the scaffold via at least three oxoether linkages. In these embodiments the peptide forms two loops, subtended between the three oxoether linkages.

The compounds of the invention thus comprise, consist essentially of, or consist of, a peptide covalently bound to a molecular scaffold. The term "scaffold" or "molecular scaffold" herein refers to a chemical moiety that is bonded to the peptide at the two or more oxoether linkages in the compounds of the invention. The term "scaffold molecule" or "molecular scaffold molecule" herein refers to a molecule that is capable of being reacted with a peptide or peptide derivative to form the conjugate having oxoether bonds. Thus, the scaffold molecule has the same structure as the scaffold moiety except that respective reactive groups (such as leaving groups or -OH groups) of the molecule are replaced by oxoether bonds to the peptide in the scaffold moiety.

The molecular scaffold molecule is any molecule which is able to connect the peptide at multiple points to form two or more oxoether bonds to the peptide. It is not a cross-linker, in that it does not normally link two peptides; instead, it provides two or more attachment points for a single peptide. Preferably, the molecular scaffold molecule comprises at least three attachment points for the peptide, referred to as scaffold reactive groups. These groups are capable of reacting with (optionally activated) hydroxyl groups on the peptide to form oxoether bonds. Thus, the molecular scaffold represents the scaffold moiety up to but not including the oxygen of the oxoether linkages in the conjugates of the invention. The scaffold molecule has the structure of the scaffold, but with reactive groups at the locations of the oxoether bonds in the conjugate of the invention. The scaffold and/or the scaffold molecule suitably has a molecular weight of less than about 1000 daltons, in some cases less than about 500 daltons, for example less than about 300 daltons.

Suitably, the scaffold comprises, consists essentially of, or consists of a (hetero)aromatic or (hetero)alicyclic moiety.

As used herein, "(hetero)aryl" is meant to include aromatic rings, for example, aromatic rings having from 4 to 12 members, such as phenyl rings. These aromatic rings can optionally contain one or more heteroatoms (e.g., one or more of N, O, S, and P), such as thienyl rings, pyridyl rings, and furanyl rings. The aromatic rings can be optionally substituted. "(hetero)aryl" is also meant to include aromatic rings to which are fused one or more other aryl rings or non-aryl rings. For example, naphthyl groups, indole groups, thienothienyl groups, dithienothienyl, and 5,6,7,8-tetrahydro-2-naphthyl groups (each of which can be optionally substituted) are aryl groups for the purposes of the present application. As indicated above, the aryl rings can be optionally substituted. Suitable substituents include alkyl groups (which can optionally be substituted), other aryl groups (which may themselves be substituted), heterocyclic rings (saturated or unsaturated), alkoxy groups (which is meant to include aryloxy groups (e.g., phenoxy groups)), hydroxy groups, aldehyde groups, nitro groups, amine groups (e.g., unsubstituted, or mono- or di-substituted with aryl or alkyl groups), carboxylic acid groups, carboxylic acid derivatives (e.g., carboxylic acid esters, amides, etc.), halogen atoms (e.g., Cl, Br, and I), and the like.

As used herein, "(hetero)alicyclic" refers to a homocyclic or heterocyclic saturated ring. The ring can be unsubstituted, or it can be substituted with one or more substituents. The substituents can be saturated or unsaturated, aromatic or nonaromatic, and examples of suitable substituents include those recited above in the discussion relating to substituents on alkyl and aryl groups. Furthermore, two or more ring substituents can combine to form another ring, so that "ring", as used herein, is meant to include fused ring systems.

Suitably, the scaffold comprises a *tris*-substituted (hetero)aromatic or (hetero)alicyclic moiety, for example a tris-methylene substituted (hetero)aromatic or (hetero)alicyclic moiety. The (hetero)aromatic or (hetero)alicyclic moiety is suitably a six-membered ring structure, preferably *tris*-substituted such that the scaffold has a 3-fold symmetry axis.
In embodiments, the scaffold is a *tris*-methylene (hetero)aryl moiety, for example a 1,3,5-*tris* methylene benzene moiety. In these embodiments, the corresponding scaffold molecule suitably has -OH or a leaving group on the methylene carbons. In these methylene substituted (hetero)aromatic compounds, the electrons of the aromatic ring can stabilize the transition state during nucleophilic substitution. Thus, for example, benzyl halides are 100-1000 times more reactive towards nucleophilic substitution than alkyl halides that are not connected to a (hetero)aromatic group.

In these embodiments the scaffold and scaffold molecule have the general formula:

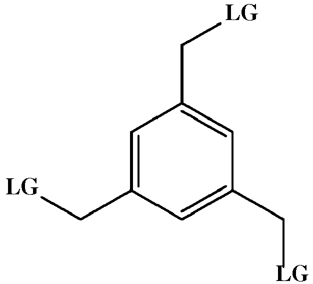

Where LG represents an -OH or a leaving group as described further below for the scaffold molecule, or LG represents the oxoether linkage to the peptide in the conjugates of the invention.

In embodiments, the group LG above may be a halogen such as, but not limited to, a bromine atom, in which case the scaffold molecule is 1,3,5-Tris(bromomethyl)benzene (TBMB). Another suitable molecular scaffold molecule is 2,4,6-Tris(bromomethyl)mesitylene. It is similar to 1,3,5-Tris(bromomethyl) benzene but contains additionally three methyl groups attached to the benzene ring. In the case of this scaffold, the additional methyl groups may form further contacts with the peptide and hence add additional structural constraint. Thus, a different diversity range is achieved than with 1,3,5-Tris(bromomethyl)benzene.

In other embodiments, the scaffold molecule may be a (hetero)aromatic or (hetero)alicyclic moiety substituted with two or more acryloyl groups, such as acrylamide or acrylate groups. These groups can undergo α,β-addition reactions with -OH to form oxoether bonds. A typical scaffold molecule of this type is 1,3,5-triacryloyl-1,3,5-triazinane (TATA) (Shen et al, (2014) Angew Chem Int Ed Engl. 2014 Feb 3;53(6):1602-6):

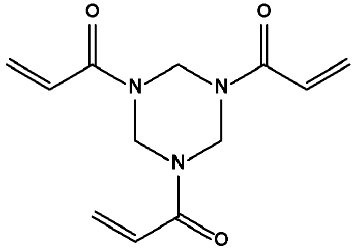

In yet other embodiments the molecular scaffold may have a tetrahedral geometry such that reaction of four functional groups of the encoded peptide with the molecular scaffold generates not more than two product isomers. Other geometries are also possible; indeed, an almost infinite number of scaffold geometries is possible, leading to greater possibilities for peptide derivative diversification.

Typically, the groups forming oxoether bonds are present on amino acid side chains on the peptide. The groups are suitably primary, secondary or tertiary alcohol groups. Preferred are the primary -CH₂OH groups of serine, threonine and suitable artificial amino acids such as N-methyl serine or Ca-substituted serines, or analogues with longer hydroxyl-containing side chains. The structure of serine is analogous to that of cysteine that has been used to form thioether bonds to the scaffold in the prior art:

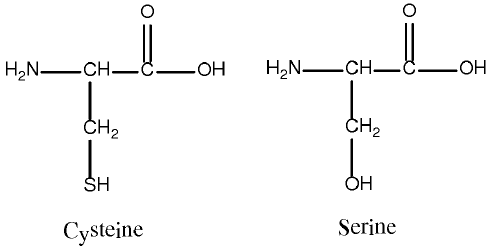

The term "oxoether" is used herein in its normal chemical sense to denote a group formed by dehydration coupling of two primary, secondary or tertiary alcohols. Oxoether groups thus consist of an oxygen atom bonded to two carbon atoms, wherein the carbon atoms are independently selected from alkyl, alkylene, or aryl carbon atoms. Suitably, the oxoether linkages of the invention comprise an oxygen atom bonded to two saturated carbon atoms, most suitably methylene (-CH₂-) carbon atoms.

The peptide element of the peptide conjugate of the invention comprises two or more reactive groups, preferably three or more reactive groups, which are responsible for binding to the scaffold; and loop amino acid sequences between the reactive groups. Diversity can be obtained, as in the prior art, by varying the sequence of the loops. The reactive group for conjugating the peptide to the molecular scaffold is, in one embodiment, -OH or a functionalised -OH group. A preferred amino acid having such a group is serine.

A particular advantage of the peptide conjugates of the invention is that they are smaller than e.g. immunological binding agents of the prior art. Typically, a compound of the present invention has a molecular weight of less than about 5000 Dalton; preferably less than about 4000 Dalton; and preferably less than about 3000 Dalton. It will be understood that a derivative constructed with multiple peptide derivatives may have a higher molecular weight outside these ranges. Moreover, peptide derivatives bound to molecules such as HSA or polyethyleneglycol (PEG) polymers will have a much higher molecular weight.

The small size of the compounds of the invention results from the use of small molecular scaffolds, typically up to about 500 Dalton in mass. The peptide itself is preferably less than about 27 amino acids in length, as measured between the N-terminal and C-terminal attachment points which attach it to the molecular scaffold. Further peptides may, of course, be present or be attached outside of the attachment points, lengthening the peptide structure. Each loop of the peptide is preferably between 0 and 9 amino acids in length, measured between adjacent attachment points. Advantageously, the loops in any peptide derivative are independently 3, 4, 5, 6, 7, 8 or 9 amino acids in length.

Several conjugated peptides may be incorporated together into the same molecule according to the present invention. For example two such peptide conjugates of the same specificity can be linked together via the molecular scaffold, increasing the avidity of the derivative for its targets. Alternatively, in another embodiment a plurality of peptide conjugates are combined to form a multimer. For example, two different peptide conjugates are combined to create a multispecific molecule. Alternatively, three or more peptide conjugates, which may be the same or different, can be combined to form multispecific derivatives. In one embodiment multivalent complexes may be constructed by linking together the molecular scaffolds, which may be the same or different.

The peptide comprises a molecular scaffold binding segment. This is the region to which the molecular scaffold is attached. Suitably the commentary regarding reactive groups on the peptide is applied to this binding segment. Suitably the molecular scaffold binding segment of the target peptide comprises 1 to 27 amino acid residues, suitably 5 to 20 amino acid residues. Suitably the molecular scaffold binding segment of the target peptide comprises fewer than 10 amino acids. This has the advantage of imposing further conformational constraint onto the peptide segment when it is attached to the molecular scaffold.

The target peptide suitably comprises the sequence (X)₁Y(X)ₘY(X)ₙY(X)ₒ, wherein Y represents an amino acid with a reactive group (such as serine), X represents a random amino acid, m and n are numbers between 1 and 20 defining the length of intervening peptide segments and 1 and o are numbers between 0 and 20 defining the length of the flanking peptide segments.

In embodiments, the peptide suitably comprises the sequence XS(X)₆S(X)₆SX, wherein X stands for a random natural or non-natural amino acid and S stands for serine, threonine or any other amino acid having primary, secondary, or tertiary alcoholic -OH in a side chain thereof, as discussed herein. In other embodiments, the peptide derivative of the invention may comprise a peptide with the sequence S(X)₆S(X)₆S

In a second aspect, the present invention provides a method of making a compound comprising at least one looped peptide structure attached via at least two oxoether linkages to a scaffold, the method comprising: providing a peptide having at least two amino residues having at least one -OH group in a side chain thereof; providing a scaffold molecule having at least two reactive sites for forming oxoether linkages with the amino residues having at least one -OH group in a side chain thereof; and forming said oxoether linkages between the peptide and the scaffold molecule.

Suitably, the methods according to this aspect of the invention are directed to making compounds according to the first aspect of the invention. Accordingly, the details of the scaffold molecule and the peptide are suitably as described above in relation to the first aspect of the invention.

The peptides for use in the methods of the invention can be made using conventional solid-phase synthesis from amino acid starting materials, which may include appropriate protecting groups as described herein. These methods for making peptides are well known in the art.

Suitably, the peptide has protecting groups on nucleophilic groups other than the -OH groups intended for forming the oxoether linkages. The nucleophilicity of amino acid side chains has been subject to several studies, and listed in descending order: thiolate in cysteines, amines in Lysine, secondary amine in Histidine and Tryptophan, guanidino amines in Arginine, hydroxyls in Serine/Threonine, and finally carboxylates in aspartate and glutamate. Accordingly, it is necessary to apply protecting groups to the more nucleophilic groups on the peptide to prevent undesired side reactions with these groups.

In embodiments, the method of the invention comprises: synthesising a peptide having protecting groups on nucleophilic groups other than the -OH groups intended for forming the oxoether linkages and second protecting groups on the -OH groups intended for forming oxoether linkages, wherein the protecting groups on the -OH groups intended for forming oxoether linkages can be removed under conditions different than for the protecting groups on the other nucleophilic groups, followed by treating the peptide under conditions selected to deprotect the -OH groups intended for forming oxoether linkages without deprotecting the other nucleophilic groups. The coupling reaction to the scaffold is then performed, followed by removal of the remaining protecting groups to yield the peptide conjugate.

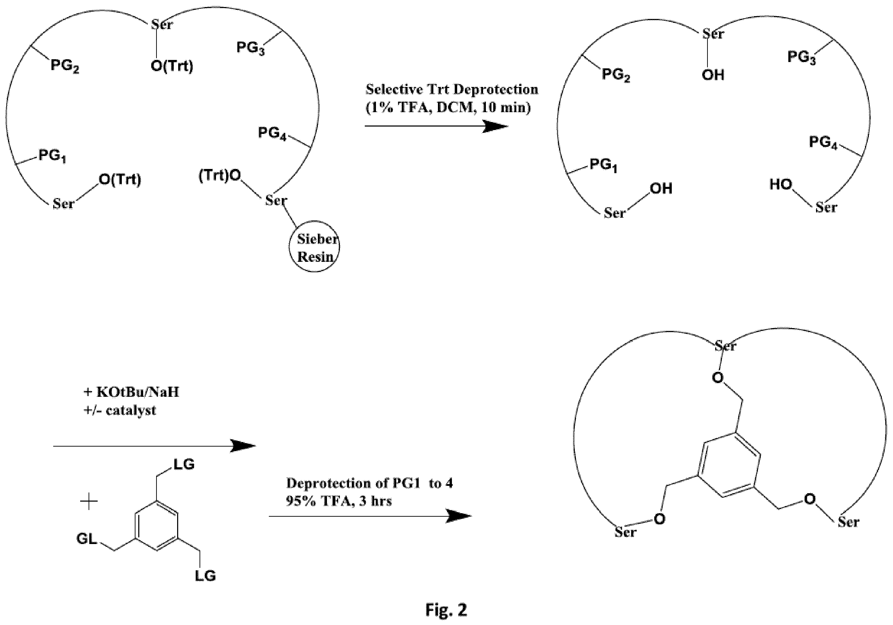

For example, the protecting groups on the -OH groups intended for forming oxoether linkages may be more acid-labile than the protecting groups on the other nucleophilic peptide residues, whereby mild acid conditions can be used to deprotect these groups without deprotecting the other nucleophilic groups. Suitably, in these embodiments, standard protecting groups commonly used in Fmoc peptide solid phase peptide synthesis are employed to make the peptide, and chosen based on acid stability such that 95% trifluoroacetic acid for about 3 hours is required to remove all protecting groups. By contrast, the -OH of serine, threonine or the like used to form the oxoether linkage is protected by a trityl group, which can readily be removed with 1% TFA in 10 minutes. The peptide is assembled on an equally acid-labile 2-chlorotrityl or Sieber resin. Following full assembly of the peptide on the solid phase, the protected peptide is selectively deprotected at the desired -OH groups using 1% TFA, and concomitantly cleaved from the resin. A method according to this embodiment is shown schematically in Fig. 2.

Suitably, the method of the invention comprises reacting, in a nucleophilic substitution reaction, the peptide having two or more -OH groups, with a scaffold molecule having two or more leaving groups.

The term "leaving group" herein is used in its normal chemical sense to mean a moiety capable of nucleophilic displacement by an alcohol -OH group. Any such leaving group can be used here provided it is readily removed by nucleophilic displacement by -OH. Suitable leaving groups are conjugate bases of acids having a pKa of less than about 5. Non-limiting examples of leaving groups useful in the invention include halo, such as bromo, chloro, iodo, O-tosylate (OTos), O-mesylate (OMes), O-triflate (OTf) or O-trimethylsilyl (OTMS).

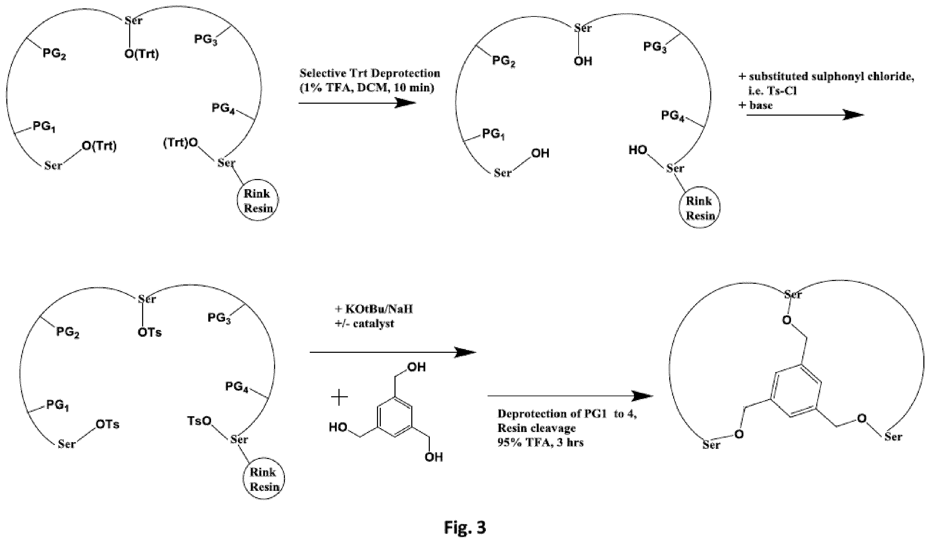

In alternative embodiments, the method comprises converting the -OH groups of the peptide intended to form oxoether linkages to leaving groups, followed by reacting the peptide, in a nucleophilic substitution reaction, with a scaffold molecule having two or more -OH groups. Conversion of the -OH groups of the peptide into leaving groups can be done in conventional fashion, such as by reaction of the hydroxyl group with respectively p-toluenesulfonic acid, methanesulfonic acid, trifluoromethanesulfonic acid, trimethylsilyl chloride or bis(trimethylsilyl)acetamide to form O-tosylate (OTos), O-mesylate (OMes), O-triflate (OTf) or O-trimethylsilyl (OTMS) leaving groups. A method according to this embodiment of the invention is shown in Fig. 2 and 3.

The nucleophilic substitution reactions may be performed in the presence of a base, for example where the leaving group is a conventional anionic leaving group. Suitable bases are sodium hydride or potassium t-butoxide, but may also constitute weaker non-nucleophilic bases such as Hunig's base, proton sponge or DBU (1,8-Diazabicycloundec-7-ene). Suitable solvents are polar, aprotic solvents such as DMF. The reaction rates can be favourably enhanced by addition of catalysts such as silver (I) oxide (Organic Syntheses, Vol. 60, p.92 (1981), A. Bouzide, G. Sauvé, Tetrahedron Lett., 1997, 38, 5945-5948), and phase exchange catalysts such as tetrabutylammonium iodide (Czernecki et al, Tetrahedron Lett., 1976, 17, 3535-3536). This ether synthesis is compatible with non-protic polar solvents such as dimethylformamide, which is desirable as polypeptides have good solubility in such solvents.

In other embodiments, the nucleophilic substitution reaction may be done under acid conditions, for example if the leaving group is 2,2,2-trichloroacetimidate and the acid is trifluoroacetic or trifluoromethane sulphonic (triflic) acid (F. Yang et al. , Tetrahedron 69 (2013) 9463-9468).

In yet other embodiments, the method comprises reacting the -OH groups on the peptide with acryloyl groups on the scaffold molecule. The acryloyl groups may, for example, be α,β-unsaturated amide or carboxylate groups on the scaffold molecule. The resulting α,β-addition reaction results in an oxoether linkage at the β-carbon atom of the acryloyl group scaffold molecule analogous to the thioether linkages to TATA described by S. Chen et al. in ChemBioChem 13, 1032-1038 (2012). These methods can be used, for example, where the scaffold molecule is TATA.

In a further aspect, the present invention provides a library comprising a plurality of different compounds according to the invention. The different compounds may comprise different peptide sequences and/or different scaffolds. The library can be screened to identify compounds having a desired biological activity.

The library may comprise or consist of, for example, at least about 10, 100 or 1000 different peptide conjugates according to the invention. Suitably, the library may consist of peptide conjugates each having the same scaffold structure but different peptides attached thereto.

In a further aspect, the present invention provides a method for selecting a candidate drug compound, comprising: providing a chemical library of compounds according to the above aspect of the invention, determining the binding of a target molecule to said compounds, and identifying a compound which maximally binds to said target molecule.

A target is a molecule or part thereof to which the peptide derivatives bind. Typically, the target will be analogous to an epitope. One skilled in the art will appreciate that the choice of target molecule is large and varied. They may be for instance human or animal proteins, cytokines, cytokine receptors, enzymes co-factors for enzymes or DNA binding proteins. Suitable cytokines and growth factors include but are not limited to: ApoE, Apo-SAA, BDNF, Cardiotrophin-1, EGF, EGF receptor, ENA78, Eotaxin, Eotaxin-2, Exodus-2, FGF-acidic, FGF-basic, fibroblast growth factor-10 (30). FLT3 derivative, Fractalkine (CX3C), GDNF, G-CSF, GM-CSF, GF-1, insulin, IFNy, IGF-1, IGF-II, IL-la, IL-1 (3, IL-2, IL-3, IL-4, IL-5, IL-6, IL-7, IL-8 (72 a.a.), IL-8 (77 a.a.), IL-9, IL-10, IL-11, IL-12, IL-13, IL-15, IL-16, IL-17, IL-17a, IL-17c,IL-17d, IL-17e, IL-17f, IL-18 (IGIF), IL-21, IL-22, IL-23, IL-31, IL-32, IL-33, IL-34, Inhibin α, Inhibin β, IP-10, keratinocyte growth factor-2 (KGF-2), KGF, Leptin, LIF, Lymphotactin, Mullerian inhibitory substance, monocyte colony inhibitory factor, monocyte attractant protein (30 ibid), M-CSF, MDC (67 a. a.), MDC (69 a. a.), MCP-1 (MCAF), MCP-2, MCP-3, MCP-4, MDC (67 a. a.), MDC (69 a. a.), MIG, MIP-1a, MIP-1p, MIP-3a, MIP3 (3, MIP-4, myeloid progenitor inhibitor factor-1 (MPIF-1), NAP-2, Neurturin, Nerve growth factor, P-NGF, NT-3, NT-4, Oncostatin M, PDGF-AA, PDGF-AB, PDGF-BB, PF-4, RANTES, SDFla, SDFlp, SCF, SCGF, stem cell factor (SCF), TARC, TGF-α, TGF-β, TGF- 2, TGF- 3, tumour necrosis factor (TNF), TNF-α, TNF-β, TNF receptor I, TNF receptor II, TNIL-1, TPO, VEGF, VEGF receptor 1, VEGF receptor 2, VEGF receptor 3, GCP-2, GRO/MGSA, GRO-β, GRO-γ, HCC1,1-309, HER 1, HER 2, HER 3 and HER 4. Cytokine receptors include receptors for the foregoing cytokines. Chemokine targets include CC chemokine derivatives CCL21/6Ckine, CCL12/MCP-5, CCL6/C10, CCL22/MDC, CCL14/HCC-1/HCC-3, CCL3L1/MIP-1 alpha Isoform LD78 beta, CCL23/Ck beta 8-1, CCL3/MIP-1 alpha, CCL28, CCL4L1/LAG-1, CCL27/CTACK, CCL4/MIP-1 beta, CCL24/Eotaxin-2/MPIF-2, CCL15/MIP-1 delta, CCL26-like/Eotaxin-3-like, CCL9/10/MIP-1 gamma, CCL26/Eotaxin-3, CCL19/MIP-3 beta, CCL11/Eotaxin,CCL20/MIP-3 alpha, CCL14a/HCC-1, CCL23/MPIF-1, CCL14b/HCC-3, CCL18/PARC, CCL16/HCC-4, CCL5/RANTES, CCL1/I-309/TCA-3, TAFA1/FAM19A1, MCK-2, TAFA5/FAM19A5, CCL2/JE/MCP-1, TAFA3/FAM19A3, CCL8/MCP-2, TAFA4/FAM19A4, CCL7/MCP-3/MARC, CCL17/TARC, CCL13/MCP-4 and CCL25/TECK; chemokine receptors include CCR1, CCR7, CCR2, CCR8, CCR3, CCR9, CCR4, CCR10, CCR5, CCRL2/LCCR/CRAM-A/B and CCR6; CXC chemokine derivatives include CXCL13/BLC/BCA-1, CXCL10/IP-10/CRG-2, CXCL14/BRAK, LIX, CXCL16, CXCL15/Lungkine, CXCL5/ENA-78, CXCL9/MIG, CXCL6/GCP-2, CXCL7/NAP-2, CXCL1/2/3/GRO, CXCL4/PF4, CXCL1/GRO alpha/KC/CINC-1, CXCL12/SDF-1 alpha, CXCL2/GRO beta/MIP-2/CINC-3, CXCL12/SDF-1 beta, CXCL3/GRO gamma/CINC-2/DCIP-1, CXCL12/SDF-1, CXCL11/I-TAC, CXCL7/Thymus Chemokine-1 and CXCL8/IL-8; CXC chemokine receptors include CXCR3, CXCR7/RDC-1, CXCR4, CXCR1/IL-8 RA, CXCR5, CXCR2/IL-8 RB and CXCR6; TNF Superfamily derivatives include 4-1BB Derivative/TNFSF9, LIGHT/TNFSF14, APRIL/TNFSF13, Lymphotoxin, BAFF/BLyS/TNFSF13B, Lymphotoxin beta/TNFSF3, CD27 Derivative/TNFSF7, OX40 Derivative/TNFSF4, CD30 Derivative/TNFSF8, TL1A/TNFSF15, CD40 Derivative/TNFSF5, TNF-alpha/TNFSF1A, EDA (pan), TNF-beta/TNFSF1B, EDA-A1/Ectodysplasin A1, TRAIL/TNFSF10, EDA-A2, TRANCE/TNFSF11, Fas Derivative/TNFSF6, TWEAK/TNFSF12 and GITR Derivative/TNFSF18; TNF Superfamily receptors include 4-1BB/TNFRSF9/CD137, NGF R/TNFRSF16, BAFF R/TNFRSF13C, Osteoprotegerin/TNFRSF11B, BCMA/TNFRSF17, OX40/TNFRSF4, CD27/TNFRSF7, RANK/TNFRSF11A, CD30/TNFRSF8, RELT/TNFRSF19L, CD40/TNFRSF5, TACI/TNFRSF13B, DcR3/TNFRSF6B, TNFRH3/TNFRSF26, DcTRAIL R1/TNFRSF23, TNF RI/TNFRSF1A, DcTRAIL R2/TNFRSF22, TNF RII/TNFRSF1B, DR3/TNFRSF25, TRAIL R1/TNFRSF10A, DR6/TNFRSF21, TRAIL R2/TNFRSF10B, EDAR, TRAIL R3/TNFRSF10C, Fas/TNFRSF6/CD95, TRAIL R4/TNFRSF10D, GITR/TNFRSF18, TROY/TNFRSF19, HVEM/TNFRSF14, TWEAK R/TNFRSF12, Lymphotoxin beta R/TNFRSF3 and XEDAR; Toll-Like Receptors including TLR-1, TLR-2, TLR-3, TLR-4, TLR-5, TLR-6, TLR-7, TLR-8 and TLR-9; enzymes, including Cathepsin A, Cathepsin B, Cathepsin C, Cathepsin D, Cathepsin E, Cathepsin F, MMP 1, MMP2, MMP 3, MMP 7, MMP 8, MMP 9, MMP 10, MMP 11, MMP 12, MMP 13, MMP 14, MMP 15, MMP 16, MMP 17, MMP 19, MMP 20, MMP 21, MMP 23A, MMP 23B, MMP 26, MMP 27, MMP 28, urokinase, kallikreins, including KLK1, KLK2, KLK3, KLK4, KLK5, KLK6, KLK7, KLK8, KLK9, KLK10, KLK11, KLK12, KLK13, KLK14 and KLK15; components of the complement system; Intracellular signalling molecules and transcription factors; p53; and MDM2. Targets may also be large plasma proteins, such as serum albumins, as set forth below.

It will be appreciated that this list is by no means exhaustive.

Additional binding or functional activities may be attached to the N or C terminus of the peptide covalently linked to a molecular scaffold. The functional group is, for example, selected from the group consisting of: a group capable of binding to a molecule which extends the half-life of the peptide derivative *in vivo,* and a molecule which extends the half-life of the peptide derivative *in vivo.* Such a molecule can be, for instance, HSA or a cell matrix protein, and the group capable of binding to a molecule which extends the half-life of the peptide derivative in vivo is an antibody or antibody fragment specific for HSA or a cell matrix protein. Such a molecule may also be a conjugate with high molecular weight PEGs.

In one embodiment, the functional group is a binding molecule, selected from the group consisting of a second peptide derivative comprising a peptide covalently linked to a molecular scaffold, and an antibody or antibody fragment. 2, 3, 4, 5 or more peptide derivatives may be joined together. The specificities of any two or more of these derivatives may be the same or different; if they are the same, a multivalent binding structure will be formed, which has increased avidity for the target compared to univalent binding molecules. The molecular scaffolds, moreover, may be the same or different, and may subtend the same or different numbers of loops.

The functional group can moreover be an effector group, for example an antibody Fc region.

Attachments to the N or C terminus may be made prior to binding of the peptide to a molecular scaffold, or afterwards. Thus, the peptide may be produced (synthetically, or by biologically derived expression systems) with an N or C terminal peptide group already in place. Preferably, however, the addition to the N or C terminus takes place after the peptide has been combined with the molecular backbone to form a conjugate. For example, Fluorenylmethyloxycarbonyl chloride can be used to introduce the Fmoc protective group at the N-terminus of the peptide. Fmoc binds to serum albumins including HSA with high affinity, and Fmoc-Trp or Fmoc-Lys bind with an increased affinity. The peptide can be synthesised with the Fmoc protecting group left on, and then coupled with the scaffold through the oxoethers. An alternative is the palmitoyl moiety which also binds HSA and has, for example been used in Liraglutide to extend the half-life of this GLP-1 analogue.

Alternatively, a conjugate of the peptide with the scaffold can be made, and then modified at the N-terminus, for example with the amine- and sulfhydryl-reactive linker N-e-maleimidocaproyloxy)succinimide ester (EMCS). Via this linker the peptide conjugate can be linked to other peptides, for example an antibody Fc fragment.

The binding function may be another peptide bound to a molecular scaffold, creating a multimer; another binding protein, including an antibody or antibody fragment; or any other desired entity, including serum albumin or an effector group, such as an antibody Fc region.

Additional binding or functional activities can moreover be bound directly to the molecular scaffold.

In embodiments, the scaffold may further comprise a reactive group to which the additional activities can be bound. Preferably, this group is orthogonal with respect to the other reactive groups on the molecular scaffold, to avoid interaction with the peptide. In one embodiment, the reactive group may be protected, and deprotected when necessary to conjugate the additional activities.

Peptide derivatives according to the present invention may be employed in *in vivo* therapeutic and prophylactic applications, *in vitro* and *in vivo* diagnostic applications, in vitro assay and reagent applications, and the like.

In general, the use of a peptide derivative can replace that of an antibody. Derivatives selected according to the invention are of use diagnostically in Western analysis and *in situ* protein detection by standard immunohistochemical procedures; for use in these applications, the derivatives of a selected repertoire may be labelled in accordance with techniques known in the art. In addition, such peptide derivatives may be used preparatively in affinity chromatography procedures, when complexed to a chromatographic support, such as a resin. All such techniques are well known to one of skill in the art. Peptide derivatives according to the present invention possess binding capabilities similar to those of antibodies, and may replace antibodies in such assays.
Diagnostic uses include any uses which to which antibodies are normally put, including test-strip assays, laboratory assays and immunodiagnostic assays.

Therapeutic and prophylactic uses of peptide derivatives prepared according to the invention involve the administration of derivatives selected according to the invention to a recipient mammal, such as a human. Substantially pure peptide derivatives of at least 90 to 95% homogeneity are preferred for administration to a mammal, and 98 to 99% or more homogeneity is most preferred for pharmaceutical uses, especially when the mammal is a human. Once purified, partially or to homogeneity as desired, the selected peptides may be used diagnostically or therapeutically (including extracorporeally) or in developing and performing assay procedures, immunofluorescent stainings and the like (Lefkovite and Pernis, (1979 and 1981) Immunological Methods, Volumes I and II, Academic Press, NY). The peptide derivatives of the present invention will typically find use in preventing, suppressing or treating inflammatory states, allergic hypersensitivity, cancer, bacterial or viral infection, and autoimmune disorders (which include, but are not limited to, Type I diabetes, multiple sclerosis, rheumatoid arthritis, systemic lupus erythematosus, Crohn's disease and myasthenia gravis).

In the instant application, the term "prevention" involves administration of the protective composition prior to the induction of the disease. "Suppression" refers to administration of the composition after an inductive event, but prior to the clinical appearance of the disease. "Treatment" involves administration of the protective composition after disease symptoms become manifest.

Animal model systems which can be used to screen the effectiveness of the peptide derivatives in protecting against or treating the disease are available.

Methods for the testing of systemic lupus erythematosus (SLE) in susceptible mice are known in the art (Knight et al. (1978) J Exp. Med., 147: 1653; Reinersten et al. (1978) New Eng. J : Med., 299: 515). Myasthenia Gravis (MG) is tested in SJL/J female mice by inducing the disease with soluble AchR protein from another species (Lindstrom et al. (1988) Adv. Inzn7unol., 42: 233). Arthritis is induced in a susceptible strain of mice by injection of Type II collagen (Stuart et al. (1984) Ann. Rev. Immunol., 42: 233). A model by which adjuvant arthritis is induced in susceptible rats by injection of mycobacterial heat shock protein has been described (Van Eden et al. (1988) Nature, 331: 171). Thyroiditis is induced in mice by administration of thyroglobulin as described (Maron et al. (1980) J. Exp. Med., 152: 1115). Insulin dependent diabetes mellitus (IDDM) occurs naturally or can be induced in certain strains of mice such as those described by Kanasawa et al. (1984) Diabetologia, 27: 113. EAE in mouse and rat serves as a model for MS in human. In this model, the demyelinating disease is induced by administration of myelin basic protein (see Paterson (1986) Textbook of Immunopathology, Mischer et al., eds. , Grune and Stratton, New York, pp. 179-213; McFarlin et al. (1973) Science, 179: 478: and Satoh et al. (1987) J ; Immunol., 138: 179).

Generally, the present peptide derivatives will be utilised in purified form together with pharmacologically appropriate carriers. Typically, these carriers include aqueous or alcoholic/aqueous solutions, emulsions or suspensions, any including saline and/or buffered media. Parenteral vehicles include sodium chloride solution, Ringer's dextrose, dextrose and sodium chloride and lactated Ringer's. Suitable physiologically-acceptable adjuvants, if necessary to keep a peptide complex in suspension, may be chosen from thickeners such as carboxymethylcellulose, polyvinylpyrrolidone, gelatin and alginates.

Intravenous vehicles include fluid and nutrient replenishers and electrolyte replenishers, such as those based on Ringer's dextrose. Preservatives and other additives, such as antimicrobials, antioxidants, chelating agents and inert gases, may also be present (Mack (1982) Remington's Pharmaceutical Sciences, 16th Edition).

The peptide derivatives of the present invention may be used as separately administered compositions or in conjunction with other agents. These can include antibodies, antibody fragments and various immunotherapeutic drugs, such as cyclosporine, methotrexate, adriamycin or cisplatinum, and immunotoxins. Pharmaceutical compositions can include "cocktails" of various cytotoxic or other agents in conjunction with the selected antibodies, receptors or binding proteins thereof of the present invention, or even combinations of selected peptides according to the present invention having different specificities, such as peptides selected using different target derivatives, whether or not they are pooled prior to administration.

The route of administration of pharmaceutical compositions according to the invention may be any of those commonly known to those of ordinary skill in the art. For therapy, including without limitation immunotherapy, the selected antibodies, receptors or binding proteins thereof of the invention can be administered to any patient in accordance with standard techniques. The administration can be by any appropriate mode, including parenterally, intravenously, intramuscularly, intraperitoneally, transdermally, via the pulmonary route, or also, appropriately, by direct infusion with a catheter. The dosage and frequency of administration will depend on the age, sex and condition of the patient, concurrent administration of other drugs, counter-indications and other parameters to be taken into account by the clinician.

The peptide derivatives of this invention can be lyophilised for storage and reconstituted in a suitable carrier prior to use. This technique has been shown to be effective and art-known lyophilisation and reconstitution techniques can be employed. It will be appreciated by those skilled in the art that lyophilisation and reconstitution can lead to varying degrees of activity loss and that use levels may have to be adjusted upward to compensate.

The compositions containing the present peptide derivatives or a cocktail thereof can be administered for prophylactic and/or therapeutic treatments. In certain therapeutic applications, an adequate amount to accomplish at least partial inhibition, suppression, modulation, killing, or some other measurable parameter, of a population of selected cells is defined as a "therapeutically-effective dose". Amounts needed to achieve this dosage will depend upon the severity of the disease and the general state of the patient's own immune system, but generally range from 0.005 to 5.0 mg of selected peptide derivative per kilogram of body weight, with doses of 0.05 to 2.0 mg/kg/dose being more commonly used. For prophylactic applications, compositions containing the present peptide derivatives or cocktails thereof may also be administered in similar or slightly lower dosages.

A composition containing a peptide derivative according to the present invention may be utilised in prophylactic and therapeutic settings to aid in the alteration, inactivation, killing or removal of a select target cell population in a mammal. In addition, the selected repertoires of peptides described herein may be used extracorporeally or in vitro selectively to kill, deplete or otherwise effectively remove a target cell population from a heterogeneous collection of cells. Blood from a mammal may be combined extracorporeally with the selected peptide derivatives whereby the undesired cells are killed or otherwise removed from the blood for return to the mammal in accordance with standard techniques.

The invention is further described with reference to the following examples.

### Example 1.

The Bicyclic Peptides chosen for comparison of thioether to oxo-ether scaffold linkage were based on 17-69-07-N241, which is a thioether-forming peptide and has sequence: H-(b-Ala)-Sar10-Ala-Cys-(D-Ala)-Asn-Glu-(1Nal)-(D-Ala)-Cys-Glu-Asp-Phe-Tyr-Asp-(tBuGly)-Cys-NH2
wherein Sar is sarcosine, D-Ala is D-Alanine, 1NAl is 1-naphthyl-L-alanine, tBuGly is α-t-butylglycine and b-Ala is β-Alanine.

The corresponding oxoether- forming Bicyclic Peptide is termed 17-69-07-N341, and its linear sequence is:
H-(b-Ala)-Sar10-Ala-Ser-(D-Ala)-Asn-Glu-(1Nal)-(D-Ala)-Ser-Glu-Asp-Phe-Tyr-Asp-(tBuGly)-Ser-NH2

Synthesis was performed with regular Fmoc amino acids using Sieber amide resin, and the following protecting groups were employed:
Boc-(b-Ala)-Sar10-Ala-Ser(Trt)-(D-Ala)-Asn(Trt/Mbh/Tmob)-Glu(OtBu)-(1Nal)-(D-Ala)-Ser(Trt)-Glu(OtBu)-Asp(OtBu)-Phe-Tyr(tBu)-Asp(OtBu)-(tBuGly)-Ser(Trt)-NH2

Following Fmoc solid phase peptide synthesis using a Symphony X instrument from Protein Technologies, the trityl protecting groups were removed from the serines with 1% TFA in dichloromethane, and peptide was concomitantly cleaved from the resin.

Solvents were evaporated under vacuum, and 0.8-1.6 of equivalents peptide was then reacted with 10 or more equivalents potassium tert-butoxide, and 1 equivalent 1,3,5 tris-bromomethylbenzene, in the presence or absence of the catalyst tetrabutylammonium iodide at 10 mole %, using anhydrous dimethylformamide as solvent.

The reaction was followed by Liquid Chromatography and mass spectrometry (LC/MS), and following consumption of the partially protected linear peptide, the partially protected cyclised peptide product was isolated.

The crude, partially protected peptide was then fully deprotected with 95% trifluoroacetic acid containing 5% standard scavengers, precipitated with diethylether, dried and purified on C18 reverse phase system. LCMS and MALDI Tof analysis showed formation of the correct product, which, at 48 Da less than 17-69-07-N241, has a mass of 2610 Da.

### Example 2

The method of Example 1 was repeated, with use of silver (I) oxide as a catalyst in the nucleophilic substitution step.

The Oxo-ether Bicyclic Peptide is termed 17-69-07-N341, and its linear sequence is:
H-(b-Ala)-Sar10-Ala-Ser-(D-Ala)-Asn-Glu-(1Nal)-(D-Ala)-Ser-Glu-Asp-Phe-Tyr-Asp-(tBuGly)-Ser-NH2

Synthesis was performed with regular Fmoc amino acids using Sieber amide resin, and the following protecting groups were employed:
Boc-(b-Ala)-Sar10-Ala-Ser(Trt)-(D-Ala)-Asn(Trt/Mbh/Tmob)-Glu(OtBu)-(1Nal)-(D-Ala)-Ser(Trt)-Glu(OtBu)-Asp(OtBu)-Phe-Tyr(tBu)-Asp(OtBu)-(tBuGly)-Ser(Trt)-NH2

Following Fmoc solid phase peptide synthesis using a Symphony X instrument from Protein Technologies, the trityl protecting groups were removed from the serines with 1 % TFA in dichloromethane, and peptide was concomitantly cleaved from the resin.

Solvents were evaporated under vacuum, and 0.8-1.6 of equivalents peptide was then reacted with 1 equivalent 1,3,5 tris-bromomethylbenzene, in the presence of the catalyst silver (I) oxide at 10 equivalents and up to 100 eq. of tetrabutylammonium iodide using anhydrous dichloromethane or dimethylacetamide as solvents.

The reaction was followed by Liquid Chromatography and mass spectrometry (LC/MS), and following consumption of the partially protected linear peptide, the partially protected cyclised peptide product was isolated.

The crude, partially protected peptide was then fully deprotected with 95% trifluoroacetic acid containing 5% standard scavengers, precipitated with diethylether, dried and purified on C18 reverse phase system. LCMS and MALDI Tof analysis showed formation of the correct product, which, at 48 Da less than 17-69-07-N241, has a mass of 2610 Da.

### Example 3

### Synthesis of oxo-ether bonded Bicyclic Peptide using 2,2,2-trichloroacetimidates in acidic conditions

The Bicyclic Peptide 17-69-07-N241 was cyclised according to the general method shown in Fig. 2, using trichloroacetimidates as leaving groups on the scaffold. Formation of the activated scaffold compound was achieved according to methodology described in Patil ((1996) Tet Letters, 37, 9, 1481-1484), to form a structure where LG is R-O-C(=NH)-CCl₃, such that it forms the tris-2,2,2 trichloro acetimidate derivative of 1,3,5 trismethanolbenzene:

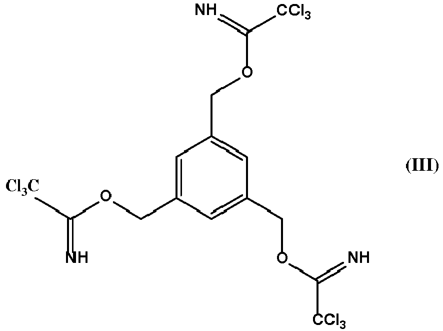

Reaction of the above compound of formula III with the partially protected linear peptide having deprotected -OH groups on the amino acids intended for linking as described in Example 1 is performed in the presence of triflic acid, and completion of the formation of the cyclised peptide is followed by mass spectrometry as previously described.

### Example 4

### Synthesis of oxo-ether bonded Bicyclic Peptide using Peptide Activation with Sulfonic Acid Esters and 1,3,5-trismethanolbenzene

A bicyclic peptide was prepared according to the scheme shown in Fig. 3. Synthesis was performed with regular Fmoc amino acids using Rink amide resin, and the following peptide sequence and protecting groups were employed:
Boc-(b-Ala)-Sar10-Ala-Ser(Trt)-(D-Ala)-Asn(Trt/Mbh/Tmob)-Glu(OtBu)-(1Nal)-(D-Ala)-Ser(Trt)-Glu(OtBu)-Asp(OtBu)-Phe-Tyr(tBu)-Asp(OtBu)-(tBuGly)-Ser(Trt)-NH2

Following Fmoc solid phase peptide synthesis using a Symphony X instrument from Protein Technologies, the trityl protecting groups were removed from the serines with 1 % TFA in dichloromethane, while peptide remained connected to the rink resin.

Activation of the three deprotected serines was performed with 20 equivalents tosyl chloride over peptide in the presence of diisopropylethylamine as base. After 3 or more hours, excess reagents and solvents were drained off. The activated peptide was then reacted with 1 equivalent of scaffold 1,3,5 tris-methanolbenzene, in the presence of base. The reaction was followed by deprotection of small quantities of resin, and assessed by LC/MS. Following completion of cyclisation, the oxo-ether bonded peptide is fully deprotected and released from the resin through acid cleavage with 95% TFA for 3 hours, and isolated.

All publications mentioned in the above specification are herein incorporated by reference. Various modifications and variations of the described aspects and embodiments of the present invention will be apparent to those skilled in the art without departing from the scope of the present invention. Although the present invention has been described in connection with specific preferred embodiments, it should be understood that the invention as claimed should not be unduly limited to such specific embodiments. Indeed, various modifications of the described modes for carrying out the invention which are apparent to those skilled in the art are intended to be within the scope of the following claims.

## Claims

1. A compound comprising at least one looped peptide structure attached via at least two oxoether linkages to a scaffold.

2. A compound according to claim 1, wherein the looped peptide structure is attached to the scaffold via at least three oxoether linkages.

3. A compound according to any preceding claim, wherein the scaffold comprises an (hetero)aromatic or (hetero)alicyclic moiety.

4. A compound according to claim 3, wherein the scaffold comprises a *tris*-substituted six-membered ring structure, preferably wherein the scaffold has a 3-fold symmetry axis.

5. A compound according to claim 3 or 4, wherein the scaffold comprises oxoether linkages at benzylic locations of the (hetero)aryl scaffold.

6. A compound according to claim 3 or 4, wherein the scaffold comprises oxoether linkages at alpha or beta- carbon positions with respect to a carbonyl, situated on a (hetero)aryl scaffold or (hetero)alicyclic scaffold

7. A compound according to any preceding claim, wherein the oxoether linkages are made through amino acid residues of the peptide having at least one -OH group in a side chain thereof, preferably wherein the oxoether linkages are made through the side-chain-OH of serine residues.

8. A method of making a compound comprising at least one looped peptide structure attached via at least two oxoether linkages to a scaffold, the method comprising:
providing a peptide having at least two amino acid residues having at least one -OH group in a side chain thereof;
optionally converting said -OH groups to leaving groups;
providing a scaffold molecule having at least two reactive sites for forming oxoether linkages with the said at least two amino acid residues; and
forming said oxoether linkages between the peptide and the scaffold molecule.

9. A method according to claim 8, wherein said peptide has protecting groups on nucleophilic groups belonging to residues other than the amino acid residues intended for forming the oxoether linkages.

10. A method according to claim 9, wherein the method comprises synthesising a peptide having first protecting groups on nucleophilic groups other than those intended for forming the oxoether linkages and second protecting groups on the -OH groups intended for forming the oxoether linkages, followed by treating the peptide under conditions selected to orthogonally deprotect the -OH groups intended for forming the oxoether linkages without deprotecting the other nucleophilic groups.

11. A method according to claim 10, wherein the protecting groups on the -OH groups intended for forming the oxoether linkages are more acid-labile than the protecting groups on the other nucleophilic groups of the peptide.

12. A method according to any of claims 8 to 11, wherein the -OH groups used to form the oxoether linkages are side chain -OH groups of serine, threonine or N-methylserine, preferably serine.

13. A method according to any of claims 9 to 11, wherein the method comprises reacting, in a nucleophilic substitution reaction, the deprotected -OH groups of the peptide with a scaffold molecule having two or more leaving groups.

14. A method according to any of claims 9 to 11, wherein the method comprises converting the deprotected -OH groups on the peptide to leaving groups, followed by reacting the peptide in a nucleophilic substitution reaction with a scaffold molecule having two or more OH groups.

15. A method according to claim 8 or 9, wherein the method comprises reacting the amino acid -OH groups with halomethyl-aryl, acryloyl or haloacetyl groups on the scaffold molecule.

16. A method according to claim 13 or 14, wherein the reaction is performed in the presence of a non-nucleophilic base and optionally also in the presence of a catalyst such as silver (I) oxide or a phase transfer catalyst such as tetrabutylammonium iodide.

17. A library comprising a plurality of different compounds according to claim 1.

18. A method for selecting a candidate drug compound, comprising: providing a library of compounds according to claim 17, determining the binding of a target molecule to said compounds, and identifying a compound which maximally binds to said target molecule.
